(19) Europäisches Patentamt / European Patent Office / Office européen des brevets

(11) **EP 3 827 085 B1**

(12) **EUROPÄISCHE PATENTSCHRIFT**

(45) Veröffentlichungstag und Bekanntmachung des Hinweises auf die Patenterteilung:
**03.12.2025   Patentblatt 2025/49**

(21) Anmeldenummer: **18745902.9**

(22) Anmeldetag: **24.07.2018**

(51) Internationale Patentklassifikation (IPC):
*C12N 15/67* (2006.01)   *C12N 15/70* (2006.01)

(52) Gemeinsame Patentklassifikation (CPC):
**C12N 15/67; C12N 15/70**

(86) Internationale Anmeldenummer:
**PCT/EP2018/069984**

(87) Internationale Veröffentlichungsnummer:
**WO 2020/020438 (30.01.2020 Gazette 2020/05)**

(54) **NEUE BAKTERIELLE LPP-MUTANTE UND DEREN VERWENDUNG ZUR SEKRETORISCHEN HERSTELLUNG VON REKOMBINANTEN PROTEINEN**

NEW BACTERIAL LPP-MUTANT AND ITS USE FOR SECRETORY PRODUCTION OF RECOMBINANT PROTEINS

NOUVEAU MUTANT LPP BACTÉRIEN ET SON UTILISATION DANS LA PRODUCTION SECRÉTRIQUE DE PROTÉINES RECOMBINANTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**02.06.2021   Patentblatt 2021/22**

(73) Patentinhaber: **Wacker Chemie AG**
**81671 München (DE)**

(72) Erfinder:
• **DASSLER, Tobias**
  **81825 München (DE)**
• **KUJAU, Marian**
  **07745 Jena (DE)**

(74) Vertreter: **Belz, Ferdinand et al**
**Wacker Chemie AG**
**Gisela-Stein-Straße 1**
**81671 München (DE)**

(56) Entgegenhaltungen:
EP-A1- 1 905 839      EP-A1- 2 204 441
EP-B1- 1 905 839      CN-A- 102 827 860
US-A1- 2008 254 511

• **ZÜCKERT WOLFRAM R ED - HAIECH JACQUES ET AL: "Secretion of Bacterial Lipoproteins: Through the Cytoplasmic Membrane, the Periplasm and Beyond", BIOCHIMICA ET BIOPHYSICA ACTA. MOLECULAR CELL RESEARCH, vol. 1843, no. 8, 26 April 2014 (2014-04-26), pages 1509 - 1516, XP028854212, ISSN: 0167-4889, DOI: 10.1016/ J.BBAMCR.2014.04.022**
• **CHOU Z GIAM ET AL: "THE JOURNAL OF BIOLOGICAL CHEMISTRY Characterization of a Novel Lipoprotein Mutant in Escherichia coli*", 10 March 1984 (1984-03-10), pages 560 - 5605, XP055542129, Retrieved from the Internet <URL:http://www.jbc.org/content/259/9/5601.full. pdf>**
• **TING-WEI CHANG ET AL: "Outer Membrane Lipoprotein Lpp Is Gram-negative Bacterial Cell Surface Receptor for Cationic Antimicrobial Peptides", JOURNAL OF BIOLOGICAL CHEMISTRY, vol. 287, no. 1, 2 January 2012 (2012-01-02), US, pages 418 - 428, XP055542076, ISSN: 0021-9258, DOI: 10.1074/jbc.M111.290361**

- **J. SHA ET AL: "Braun Lipoprotein (Lpp) Contributes to Virulence of Yersiniae: Potential Role of Lpp in Inducing Bubonic and Pneumonic Plague", INFECTION AND IMMUNITY, vol. 76, no. 4, 28 January 2008 (2008-01-28), US, pages 1390 - 1409, XP055542080, ISSN: 0019-9567, DOI: 10.1128/IAI.01529-07**

Bemerkungen:

Das gesamte Dokument mit Referenztabelle(n) und Sequenzliste(n) kann von der Webseite des EPA heruntergeladen werden

Bemerkungen:

Das gesamte Dokument mit Referenztabelle(n) und Sequenzliste(n) kann von der Webseite des EPA heruntergeladen werden

**Beschreibung**

Die Erfindung betrifft eine neuartige bakterielle lpp-Mutante und deren Verwendung in einem Fermentationsverfahren zur sekretorischen Herstellung von rekombinanten Proteinen.

Der Markt für rekombinante Proteinpharmazeutika (Pharmaproteine/Biologics) ist in den letzten Jahren stark gewachsen. Besonders wichtige Proteinpharmazeutika sind eukaryontische Proteine, vor allem Säugetierproteine und menschliche Proteine. Beispiele wichtiger Pharmaproteine (pharmazeutisch aktive Proteine) sind Cytokine, Wachstumsfaktoren, Proteinkinasen, Protein- und Peptidhormone sowie Antikörper und Antiköperfragmente. Aufgrund der immer noch sehr hohen Produktionskosten für Pharmaproteine wird laufend nach effizienteren und damit kostengünstigeren Verfahren und Systemen für deren Herstellung gesucht.

Generell werden rekombinante Proteine entweder in Säugerzellkulturen oder in mikrobiellen Systemen hergestellt. Mikrobielle Systeme weisen gegenüber Säugerzellkulturen den Vorteil auf, dass auf diese Weise rekombinante Proteine in kürzerer Zeit und zu geringeren Kosten hergestellt werden können. Für die Produktion von rekombinanten Proteinen eignen sich daher vor allem Bakterien. Aufgrund seiner sehr gut untersuchten Genetik und Physiologie, der kurzen Generationszeit und der einfachen Handhabung, ist das Gram-negative Enterobakterium *Escherichia coli* (*E.* coli) gegenwärtig der am häufigsten verwendete Organismus zur Produktion rekombinanter Proteine. Besonders attraktiv sind Produktionsverfahren, bei denen das Zielprotein von den Bakterienzellen in korrekter Faltung direkt ins Fermentationsmedium abgegeben wird, da auf diese Weise ein aufwendiger Zellaufschluss und gegebenenfalls ein verlustreicher Proteinrückfaltungsprozess entfällt. Ein weiterer Vorteil der extrazellulären Produktion liegt darin, dass durch die Freisetzung des Zielproteins in das Kulturmedium oftmals die Produkt-Ausbeute gesteigert werden kann, da die Anhäufung des Zielproteins nicht auf den Raum des Periplasmas bzw. des Cytoplasmas beschränkt ist.

Für die extrazelluläre Produktion eines Zielproteins sind beispielsweise sogenannte "leaky"-Stämme von *E. coli* geeignet, die aufgrund des Fehlens oder der Veränderung von bestimmten Strukturelementen der Zellhülle im Periplasma befindliche Proteine vermehrt in das Medium entlassen. Derartige "leaky"-Stämme können veränderte Lipoprotein-Anteile in der äußeren Membran aufweisen, wie es u.a. bei bestimmten Mutanten im Braun'sche Lipoprotein (lpp) der Fall ist (Inouye et al. 1977, J. Bact. 132, S. 308-313; Suzuki 1978, Mol. Gen. Genet. 167, S. 1-9; Giam et al. 1984, Eur. J. Biochem. 141, S. 331-379).

Es sind Produktionsverfahren für heterologe Proteine im technischen Maßstab offenbart, bei denen mit Hilfe von unterschiedlichen lpp-Mutanten von *E. coli* eine Freisetzung von Zielproteinen ins Fermentationsmedium erreicht wird (US 2008/0254511 A1, US 5,223,482 A).

Aufgrund der höheren Neigung zur Zelllyse haben "leaky"-Stämme bei der Produktion mancher heterologer Zielproteine jedoch den Nachteil, dass sie trotz bestimmter Maßnahmen zur Stabilisierung der Zellen, wie z.B. dem Zusatz erhöhter Mengen an Ca- und Mg-Ionen zum Kulturmedium (s. US 2008/0254511 A1), relativ früh und stark lysieren, wodurch einerseits die Protein-Produktionsphase verkürzt wird und somit die Produkt-Ausbeute geringer ausfällt, als sie bei einer längeren Produktionsphase sein könnte. Und andererseits führt die Zelllyse zu einem Anstieg der Viskosität des Mediums, was vor allem auf die bei der Zelllyse freigesetzte DNS zurückzuführen ist. Dadurch wird die anschließende Aufreinigung und Gewinnung des Zielproteins erschwert, was wiederum zu unnötig hohen Prozesskosten führt. EP 2 204 441 A1 offenbart ein fermentatives Verfahren zur Herstellung eines rekombinanten Proteins mit Hilfe eines E. coli-Stamms, der aufgrund einer Mutation im spoT-Gen eine attenuierte (p)ppGpp-Synthetase II-Aktivität aufweist und zusätzlich ein mutiertes lpp-Gen enthält, was dazu führt, dass die Zellen vermehrt periplasmatische Proteine ins Medium abgeben. In EP 2 204 441 A1 ist kein Lpp-Fusionsprotein beschrieben, sondern verschiedene bekannte Mutationen im lpp-Gen (lpp1, lpp3 und ∆lpp), die einen leaky-Phänotyp und damit eine erhöhte Freisetzungsrate von rekombinanten Proteinen aus dem Periplasma in das Kulturmedium zur Folge haben.

Zückert (2014, Biochimica et Biophysica Acta 1843, S. 1509-16) beschäftigt sich mit dem Export von Prolipoproteinen durch die cytoplasmatische Membran und deren schrittweise Reifung in unterschiedlichen Mikroorganismen. Dabei werden auch verschiedene Lpp-Mutanten betrachtet, allerdings kein Lpp-Fusionsprotein, das zweimal die Aminosäuresequenz von Lpp aufweist. Auch der Einfluss, den die genannten Lipoproteine bzw. mutierte Varianten davon möglicherweise auf die Freisetzung von rekombinanten Proteinen in das Kulturmedium haben, wird in diesem Review nicht untersucht.

Giam et al. (1984, J Biol Chem 259, S. 5601-5) offenbart eine Mutante von E. coli, die ein durch einen Aminosäureaustausch (C68R) strukturell verändertes Lpp-Lipoprotein bildet. Die Publikation charakterisiert diese Lpp-Mutante und beschreibt die Kinetik seiner Reifung. Eine mögliche Rolle bei der Sekretion rekombinanter Proteine wird nicht untersucht.

Chang et al. (2012, J Biol Chem 287, S. 418-28) beschäftigt sich mit der Rolle, die das Membranprotein Lpp der äußeren Membran von Gram-negativen Bakterien als Rezeptor für antimikrobielle Peptide spielt. Es wird weder ein Lpp-Fusionsprotein, noch die Rolle, die Lipoproteine in der Sekretion rekombinanter Proteine spielen, offenbart.

Sha et al. (2008, Infect Immun 76, S. 1390-409) untersucht den Einfluss des Braun'schen Lipoproteins Lpp auf die Virulenz von Yersinia-Stämmen und arbeitet dabei unter anderem mit lpp-Deletionsmutanten. Dabei wird die Rolle, die Lipoproteine in der Sekretion rekombinanter Proteine spielen, nicht betrachtet.

CN 102 827 860 B offenbart verschiedene Doppelmutanten, in denen das lpp-Gen deletiert sein kann, die dann für die sekretorische Produktion rekombinanter Proteine eingesetzt werden. Ein Sekretionsstamm, der ein Lpp-Fusionsprotein zusätzlich zum bzw. anstelle des natürlichen Lpp-Proteins produziert, wird nicht beschrieben.

Aufgabe der Erfindung ist die Bereitstellung eines mutierten Bakterienstammes zur Verwendung in einem fermentativen Verfahren zur Herstellung eines rekombinanten Zielproteins, wobei der überwiegende Teil des Zielproteins während der Kultivierung in das Fermentationsmedium ausgeschieden wird und die im Kulturmedium vorliegende Zielprotein-Menge höher ist als bei im Stand der Technik offenbarten Bakterienstämmen, d.h. das Zielprotein soll in erhöhter Ausbeute im Kulturmedium vorliegen.

Diese Aufgabe wird gelöst durch einen Bakterienstamm enthaltend mindestens ein Gen codierend für ein rekombinantes Protein

dadurch gekennzeichnet, dass

er einen offenen Leserahmen bestehend aus

i einem DNS-Fragment codierend ein N-terminales Signalpeptid, das die Translokation des Proteins in das Periplasma vermittelt,

> wobei die Aminosäuresequenz des Signalpeptids des Lpp-Fusionsproteins identisch zur Aminosäuresequenz des Signalpeptids des Wildtyp-Lpp-Proteins (SEQ ID Nr. 2 von Aminosäure 1 bis 20) ist oder
> das N-terminale Signalpeptid an Aminosäure-Position 14 statt Glycin eine andere proteinogene Aminosäure enthält und an allen anderen Aminosäure-Positionen identisch zum Signalpeptid des Wildtyp-Lpp-Proteins ist oder
> wobei es sich beim N-terminalen Signalpeptid um ein Signalpeptid der Lipoproteine Pal, NlpI, NlpB oder OsmB von E*scherichia coli* handelt,
> verknüpft mit

ii einer daraufolgenden DNS-Sequenz *(lpp(N))* codierend ein Lipoprotein (Lpp(N)), wobei die von lpp(N) codierte Aminosäuresequenz die Aminosäuresequenz des Wildtyp-Lpp-Proteins (SEQ ID Nr. 2 von Aminosäure 21 bis 78) ist oder sich nur dadurch von dieser unterscheidet, dass in Lpp(N) die im Wildtyp-Lpp-Protein vorhandene C-terminale Aminosäure Lysin mutiert ist und

iii einer weiteren DNS-Sequenz (lpp(C)) codierend ein Lipoprotein (Lpp(C)), wobei die von lpp(C) codierte Aminosäuresequenz die Aminosäuresequenz des Wildtyp-Lpp-Proteins ist oder sich von dieser nur dadurch unterscheidet, dass in Lpp(C) die im Wildtyp-Lpp-Protein vorhandene N-terminale Aminosäure Cystein mutiert ist,

enthält.

Beim Bakterienstamm handelt es sich um einen Stamm der Art E*scherichia* coli.

Als offener Leserahmen *(open reading frame,* ORF) wird derjenige Bereich der DNS bzw. RNS bezeichnet, der zwischen einem Startcodon und einem Stoppcodon liegt und für die Aminosäuresequenz eines Proteins codiert. Der ORF wird auch als codierende Region bezeichnet.

ORFs werden von nicht-codierenden Bereichen umgeben. Als Gen wird daher der DNS-Abschnitt bezeichnet, der alle Grundinformationen zur Herstellung einer biologisch aktiven RNA enthält. Ein Gen enthält also nicht nur den DNS-Abschnitt, von dem durch Transkription eine einzelsträngige RNA-Kopie hergestellt wird, sondern auch zusätzliche DNS-Abschnitte, die an der Regulation dieses Kopiervorgangs beteiligt sind. Da ein Gen mindestens einen ORF enthält codiert ein Gen auch für mindestens ein Protein.

In den ORFs codiert jeweils ein Basentriplett der DNS eine bestimmte Aminosäure oder ein Stoppsignal. Die als Bausteine für die Bildung von Proteinen codierten Aminosäuren werden auch als proteinogene Aminosäuren bezeichnet.

Der im Rahmen dieser Erfindung in der Einzahl verwendete Begriff "ein/das rekombinante/s Protein" kann auch mehrere verschiedene rekombinante Proteine bedeuten. Bevorzugt handelt es sich um 1 bis 3 verschiedene rekombinante Proteine, besonders bevorzugt um 1 rekombinantes Protein oder 2 verschiedene rekombinante Proteine. Das rekombinante Protein wird auch als Zielprotein bezeichnet.

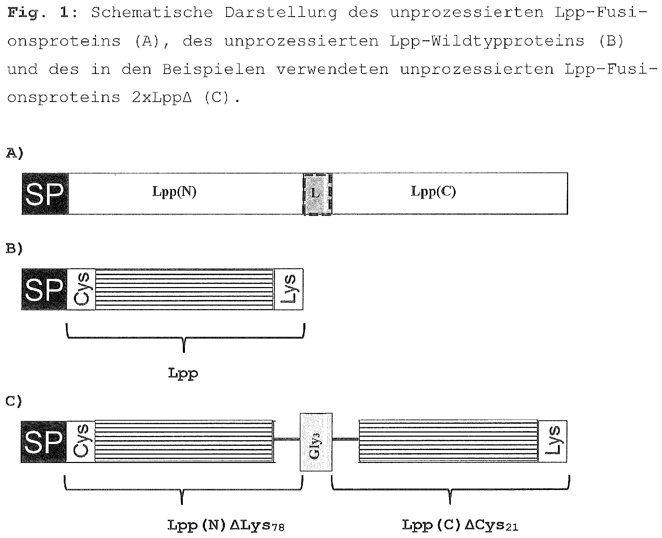

Die DNS-Sequenz des lpp-Wildtypgens von *E. coli* (SEQ ID Nr. 1, publiziert unter der EcoGene Accession No. EG10544) codiert für ein unprozessiertes Lpp-Protein (Lpp-Präprotein), das aus 78 Aminosäuren besteht (SEQ ID Nr. 2). Die ersten 60 Nukleotide codieren dabei für das Signalpeptid, welches die Sekretion des Proteins in das Periplasma steuert und das nach der Translokation abgespalten wird (Prozessierung). Am N-Terminus des prozessierten Lpp-Wildtypproteins befindet sich ein Cystein-Rest (Cys), am C-Terminus ein Lysin-Rest (Lys) (s. Fig. 1B), welche beide von der Zelle posttranslational modifiziert werden, um so die volle Funktion des Lpp-Proteins - nämlich die Verbindung der äußeren Membran mit der Peptidoglykanschicht (auch bakterielle Zellwand genannt) - zu gewährleisten (Giam et al. 1984, J. Biol. Chem. 259, S. 5601-5605). Die Begriffe Lpp, Lpp-Protein Wildtyp-Lpp-Protein und Lpp-Wildtypprotein werden in der vorliegenden Erfindung synonym verwendet und in der Fachliteratur auch als Lipoprotein, Murein Lipoprotein oder Braun'sches Lipoprotein bezeichnet. Das Protein Lpp wird vom Gen *lpp (lpp-Gen,* Wildtyp-lpp-Gen, lpp-Wildtypgen) codiert.

Der erfindungsgemäße Bakterienstamm enthält einen ORF bestehend aus dem DNS-Fragment codierend ein N-terminales Signalpeptid, *lpp(N)* und *lpp(C)*. Das von diesem ORF codierte Protein ist ein Lpp-Fusionsprotein. Unter einem Lpp-Fusionsprotein ist im Rahmen der vorliegenden Erfindung ein Fusionsprotein zu verstehen, das aus zwei Lpp-Proteinen zusammengesetzt ist (im folgenden Lpp-Anteile genannt, wobei jeder Anteil einem möglicherweise mutierten Lpp-Wildtypprotein entspricht) und das in der unprozessierten Form ein Signalpeptid (SP) trägt. In Fig. 1A ist ein derartiges Fusionsprotein in der unprozessierten Form schematisch dargestellt. Dabei ist der N-terminale Anteil (Lpp(N)), welcher in der unprozessierten Form des Fusionsproteins mit dem Signalpeptid verknüpft ist, mit einem C-terminalen Anteil (Lpp(C)) verbunden. Lpp(N) und Lpp(C) können direkt oder über eine Aminosäure-Linkersequenz (L) bestehend aus einer bis mehreren Aminosäuren verbunden sein.

Die Aminosäure-Sequenz jedes der beiden Lpp-Anteile des Fusionsproteins entspricht in einer Ausführungsform der Erfindung der prozessierten Lpp-Wildtypsequenz In der vorliegenden Erfindung wird das Lpp-Fusionsprotein auch 2xLpp-Protein genannt und vom 2xlpp-Gen codiert.

Zu den in der Aminosäure-Sequenz des Lpp-Fusionsproteins potentiell vorhandenen Mutationen zählen Substitutionen (Austausch von Aminosäuren), Deletionen (Fehlen von Aminosäuren) und Insertionen (Einfügen von zusätzlichen Aminosäuren).

Bei erfindungsgemäßen Stämmen ist das 2xlpp-Gen codierend das Lpp-Fusionsprotein entweder auf dem Chromosom oder auf einem Plasmid enthalten. Bevorzugt ist das Gen codierend das Lpp-Fusionsprotein auf dem Chromosom lokalisiert.

Bevorzugt ist der Bakterienstamm dadurch gekennzeichnet, dass er kein weiteres Gen enthält, das ein Protein mit einer Identität von mindestens 80% im Vergleich zum prozessierten Wildtyp-Lpp-Protein codiert. Die Sequenz des prozessierten Wildtyp-Lpp-Proteins ist in SEQ ID Nr. 2 von Aminosäure 21 bis Aminosäure 78 angegeben.

Besonders bevorzugt enthält der erfindungsgemäße Bakterienstamm kein Wildtyp-lpp-Gen. Bevorzugt sind also Bakterienstämme, bei denen das Lpp-Fusionsprotein die einzige Lpp-Form ist, die in diesen Stämmen vorkommt. In dieser Ausführungsform bilden die Bakterienstämme kein Lpp-Wildtypprotein und außer dem Lpp-Fusionsprotein keine durch Aminosäure-Austausche vom Lpp-Wildtypprotein abgeleitete Lpp-Variante.

Bevorzugt sind Lpp(N) und Lpp(C) über einen Linker bestehend aus einer bis mehreren, besonders bevorzugt einer bis 20 und insbesondere bevorzugt einer bis 10 Aminosäuren verbunden. Für die Linkersequenz kommen grundsätzlich alle zwanzig proteinogenen Aminosäuren in beliebiger Reihenfolge in Frage. Bevorzugte Aminosäuren, aus denen die Linkersequenz aufgebaut ist, sind Glycin, Serin und Alanin. In einer besonders bevorzugten Ausführungsform besteht die Linkersequenz aus drei Glycin-Resten.

Die von *lpp(N)* und *lpp(C)* codierten Aminosäuresequenzen unterscheiden sich in einer alternativen Ausführungsform der Erfindung dadurch von der Aminosäuresequenz des Wildtyp-Lpp-Proteins, dass

    i) Lpp(N) die im Wildtyp-Lpp-Protein vorhandene C-terminale Aminosäure Lysin mutiert ist oder
    ii) Lpp(C) die im Wildtyp-Lpp-Protein vorhandene N-terminale Aminosäure Cystein mutiert ist.

In einer besonders bevorzugten Ausführungsform ist im Unterschied zum Lpp-Wildtypprotein bei Lpp(N) der C-terminale Lysin-Rest und bei Lpp(C) der N-terminale Cystein-Rest mutiert. Insbesondere bevorzugt sind diese Reste deletiert (s. Fig. 1C). Durch diese Mutation sollen die an diesen Aminosäure-Resten normalerweise erfolgenden posttranslationalen Modifikationen verhindert werden.

In SEQ ID Nr. 3 (schematisch dargestellt in Fig. 1C) ist ein Beispiel für eine besonders bevorzugte Sequenz eines noch unprozessierten Lpp-Fusionsproteins angegeben, die ein Signalpeptid (Aminosäure 1-20), Lpp(N)$\Delta$Lys$_{78}$ (Aminosäure 21-77) und Lpp(C)$\Delta$Cys$_{21}$ (Aminosäure 81-137) enthält, wobei sich Lpp(N)$\Delta$Lys$_{78}$ nur durch die Deletion des C-terminalen Lysin-Rests und Lpp(C)$\Delta$Cys$_{21}$ nur durch die Deletion des N-terminalen Cystein-Rests jeweils von der Lpp-Wildtypsequenz unterscheiden und über eine Linkersequenz bestehend aus drei Glycin-Resten miteinander verbunden sind. Dieses Protein wird im Rahmen dieser Erfindung mit 2xLpp$\Delta$ bezeichnet. Das 2xLpp$\Delta$-Protein wird vom in SEQ ID Nr. 16 angegebenen DNS-Fragment codiert. Dieses DNS-Fragment wird mit 2x*lpp*$\Delta$ bezeichnet.

Als Signalpeptid für die Translokation des Lpp-Fusionsproteins in das Periplasma kommen prinzipiell alle Signalpeptide von Lipoproteinen aus *E. coli* in Frage. In einer Ausführungsform handelt es sich beim N-terminalen Signalpeptid um ein Signalpeptid der Lipoproteine Lpp, Pal, NlpI, NlpB oder OsmB (Hayashi und Wu 1990, J. Bioenerg. Biomembr. 22, S. 451-471).

In einer alternativen Ausführungsform weist das Signalpeptid des Lpp-Fusionsproteins die Sequenz des Signalpeptids des Wildtyp-Lpp-Proteins auf.

Die Aminosäuresequenz des Signalpeptids des Lpp-Fusionsproteins ist identisch zur Aminosäuresequenz des Signalpeptids des Wildtyp-Lpp-Proteins, insbesondere bevorzugt ist auch die Nukleotidsequenz identisch. Die Aminosäuresequenz des Signalpeptids des Wildtyp-Lpp-Proteins ist angegeben in SEQ ID Nr. 2 von Aminosäure 1 bis 20, die Nukleotidsequenz in SEQ ID Nr. 1 von Nukleotid 1 bis 60.

In einer weiteren alternativen Ausführungsform ist der Bakterienstamm dadurch gekennzeichnet, dass das N-terminale Signalpeptid an Aminosäure-Position 14 statt Glycin eine andere proteinogene Aminosäure enthält und an allen anderen Aminosäure-Positionen identisch zum Signalpeptid des Wildtyp-Lpp-Proteins ist.

Besonders bevorzugt ist der Bakterienstamm dadurch gekennzeichnet, dass es sich bei der proteinogenen Aminosäure an Position 14 des N-terminalen Signalpeptids um Asparaginsäure handelt. In diesem Fall ist an Position 14 ein Asparaginsäure-Rest anstelle des Glycin-Restes (G14D-Austausch) vorhanden und alle anderen Aminosäure-Positionen sind identisch zum Signalpeptid des Wildtyp-Lpp-Proteins.

Die Expression des 2xlpp-Gens kann durch jeden in *E. coli* funktionellen Promotor gesteuert werden. Bevorzugt sind dabei Promotoren, die unter den meisten Wachstumsbedingungen konstitutiv aktiv sind (z.B. $\sigma^{70}$-abhängige Promotoren), wie z.B. die Promotoren der Gene gapA, rpiA, mppA, lpp, catB, tufB und proC, sowie natürliche oder artifizielle Promotoren ohne regulatorisch aktive Operatorregion wie z.B. der tetA-, der lac-, der tac- und der trp-Promotor. Der Promotor kann entweder die natürliche Sequenz aufweisen oder durch Basenaustausche in seiner Stärke moduliert werden.

In einer bevorzugten Ausführungsform ist der Bakterienstamm dadurch gekennzeichnet, dass der offene Leserahmen codierend das 2xLpp-Protein im Chromosom anstelle der in SEQ ID Nr. 1 angegebenen Sequenz lokalisiert ist. In diesen Stämmen, bei denen der chromosomale lpp-Wildtyp-ORF durch den ORF für das Lpp-Fusionsprotein ersetzt worden ist, steht die Expression des Lpp-Fusionsproteins unter der Kontrolle des natürlichen lpp-Promotors. D.h. das 2xlpp-Gen steht unter der Kontrolle des Promotors, der auch für die Expression des Wildtyp-Lpp-Proteins verantwortlich ist.

Es sind dem Fachmann Methoden zur Erzeugung eines Gens für ein erfindungsgemäßes Lpp-Fusionsprotein bekannt.

Ein derartiges Gen wird in der Regel zunächst *in vitro* erzeugt und anschließend in die Zelle eingebracht. Das Gen für das Lpp-Fusionsprotein kann beispielsweise durch Verspleißung zweier DNS-Moleküle, die jeweils für einen der beiden Lpp-Teile des Fusionsproteins codieren, mit Hilfe der "Overlap Extension"-PCR-Methode hergestellt werden (Horton et al. 2013, BioTechniques 54, S. 129-133), wobei zunächst die DNS des lpp-Wildtyp-Gens als Matrize dient. Alternativ kann das 2xlpp-Gen auch komplett mittels Gensynthese erzeugt werden.

Soll das Gen für das Lpp-Fusionsprotein von einem Plasmid in der Zelle exprimiert werden, so muss das Gen zunächst in das Plasmid kloniert werden. Dafür sind alle bekannten im gewählten Bakterienstamm vermehrbaren Plasmide geeignet, wie z.B. Derivate von bekannten Expressionsvektoren wie pJF118EH, pKK223-3, pUC18, pBR322, pACYC184, pASK-IBA3 oder pET. Methoden für die Integration des 2xlpp-Gens in das Plasmid sowie für die Transformation des Plasmids in Bakterienzellen sind dem Fachmann bekannt.

Alternativ kann das *in vitro* erzeugte 2xlpp-Gen auch mittels verschiedener Standardmethoden in das Chromosom einer Wirtszelle integriert werden. Diese Integration kann entweder am natürlichen *lpp*-Genort erfolgen, wodurch das ursprünglich dort befindliche *lpp*-Wildtypgen durch das *2xl*pp-Gen ersetzt wird, oder aber an einer anderen Stelle des Chromosoms.

Die Integration in das Chromosom kann beispielsweise mittels des in Link et al. (1997, J. Bacteriol. 179, S. 6228-6237) beschriebenen Verfahrens über den Mechanismus der homologen Rekombination erfolgen. Dazu muss das Gen codierend das Lpp-Fusionsprotein zunächst in das Plasmid pKO3 kloniert werden, welches dann in die Zelle eingebracht wird. Mit diesen Transformanten wird dann die in Link *et al.* beschriebene Prozedur durchgeführt, bei der das Gen codierend das Lpp-Fusionsprotein in das Chromosom eingebaut wird.

Alternativ kann das DNS-Fragment, welches das *2xl*pp-Gen enthält, auch direkt nach der von Sun et al. beschriebenen Methode (2008, Appl. Environ. Microbiol. 74, S. 4241-4245) in die Zelle transformiert und an der gewünschten Stelle in das Chromosom integriert werden. Dabei macht man sich das Prinzip der Gegenselektion zunutze. Zuerst wird am gewünschten Genort, an dem das 2xlpp-Gen in das Chromosom der Zelle integriert werden soll, eine Expressionskassette eingebracht, die das cat-Gen, welches für eine Chloramphenicol-Acetyltransferase codiert, sowie das sacB-Gen von Bacillus subtilis, welches für die Laevansucrase codiert, enthält. Die Integration dieser Kassette ist nach der Methode von Datsenko und Wanner (2000, Proc. Natl. Acad. Sci. U S A. 97, S. 6640-6645) möglich, wobei durch Selektion auf Chloramphenicol-Resistenz korrekte Integranten identifiziert werden können. Derartige Zellen sind resistent gegenüber Chloramphenicol und aufgrund der Expression des *sacB-Gens* sensitiv gegenüber Saccharose. Anschließend werden diese Zellen dann mit einem linearen DNS-Fragment transformiert, welches das *2xl*pp-Gen enthält und dessen Flanken homologe DNS-Sequenzen zum gewünschten Genort aufweisen, um dort eine ortsspezifische Integration des DNS-Fragments zu gewährleisten. Zellen, bei denen ein Austausch der cat-sacB-Kassette gegen das 2xlpp-Gen erfolgt ist, können aufgrund der Wiederherstellung der Wachstumsfähigkeit in Gegenwart von Saccharose identifiziert werden. Die finale Überprüfung der korrekten Integration des *2xl*pp-Gens in das Chromosom kann mittels PCR unter Verwendung von für den Integrationsort spezifischen Oligonukleotiden und anschließender Sequenzierung des PCR-Produkts erfolgen.

Bevorzugt handelt es sich bei den rekombinanten Proteinen um heterologe Proteine. Unter einem heterologen Protein ist im Sinne der vorliegenden Erfindung ein Protein zu verstehen, das nicht zum Proteom, *d.h.* der gesamten natürlichen Protein-Ausstattung, des Bakterienstammes gehört.

Bevorzugt handelt es sich bei dem heterologen Protein um ein eukaryontisches Protein, besonders bevorzugt um ein Protein, welches eine oder mehrere Disulfid-Brücken enthält oder welches in seiner funktionellen Form als Di- oder Multimer vorliegt, d.h. dass das Protein eine Quartärstruktur besitzt und aus mehreren identischen (homologen) oder nicht-identischen (heterologen) Untereinheiten aufgebaut ist.

Zu den wichtigsten heterologen Proteinklassen zählen Antikörper und deren Fragmente, Cytokine, Wachstumsfaktoren, Proteinkinasen, Proteinhormone, Lipokaline, Antikaline, Enzyme, Bindeproteine und molekulare Scaffolds und davon abgeleitete Proteine und pharmakologisch wirksame Peptide. Beispiele für diese Proteinklassen sind u.a. heavy-chain Antikörper und deren Fragmente (z.B. Nanobodies), einzelkettige Antikörper, Interferone, Interleukine, Interleukinrezeptoren, Interleukinrezeptor Antagonisten, G-CSF, GM-CSF, M-CSF, Leukämieinhibitoren, Stammzellenwachstumsfaktoren, Tumornekrosefaktoren, Wachstumshormone, insulinartige Wachstumsfaktoren, Fibroblastenwachstumsfaktoren, von Blutplättchen abstammende Wachstumsfaktoren, transformierende Wachstumsfaktoren, Hepatocyten-Wachstumsfaktoren, knochenmorphogenetische Faktoren, Nervenwachstumsfaktoren, vom Gehirn abstammende neurotrophe Faktoren (BDNF), von Gliazelllinien abstammende neurotrophe Faktoren, Angiogenese-Inhibitoren, Gewebeplasminogen-Aktivatoren, Blutgerinnungsfaktoren, Trypsin-Inhibitoren, Elastase-Inhibitoren, Komplementbestandteile, hypoxie-induzierte Stressproteine, Proto-Oncogen-Produkte, Transkriptionsfaktoren, viruskonstitutive Proteine, Proinsulin, Parathormon, Prourokinase, Erythropoietin, Thrombopoietin, Neurotrophin, Protein C, Glucocerebrosidase, Superoxid-Dismutase, Renin, Lysozym, P450, Prochymosin, Lipocortin, Reptin, Serumalbumin, Streptokinase, Tenecteplase, CNTF und Cyclodextrin-Glycosyl-Transferasen.

Beispiele für von molekularen Scaffolds abgeleiteten Proteinen sind u.a. Evibodies (abgeleitet von CTLA-4), Affibodies (von Protein A von *S. aureus*), Avimere (von humaner A-Domäne Familie), Transbodies (von Transferrin), DARPins (vom Ankyrin Repeat Protein), Adnectin (von Fibronectin III), Peptid Aptamere (von Thioredoxin), Microbodies (von Microprotein), Affiline (von Ubiquitin), $\alpha$-Crystallin, Charybdotoxin, Tetranectin, PDZ Domäne des RAS-bindenden Proteins AF-6, Kunitz-Typ Domäne von Proteininhibitoren.

Eine besonders bevorzugte Klasse von Proteinen, die aus mehreren Proteinuntereinheiten besteht, sind Antikörper. Besonders bevorzugt ist der Bakterienstamm daher dadurch gekennzeichnet, dass es sich beim heterologen Protein um einen Antikörper oder ein Fragment eines Antikörpers handelt. Antikörper werden in der Forschung, in der Diagnostik und als Therapeutikum in großem Umfang eingesetzt, so dass besonders effiziente und in technischem Ausmaß mögliche Produktionsverfahren notwendig sind.

Auch funktionelle Fab-Antikörperfragmente und Volllängen-Antikörper können mittels des erfindungsgemäßen Verfahrens extrazellulär hergestellt werden. Bevorzugte Volllängen-Antikörper sind dabei Antikörper der IgG- und IgM-Klasse, insbesondere der IgG-Klasse.

Bei der Herstellung funktioneller Fab-Antikörperfragmente muss die Zelle das entsprechende Fragment der leichten Kette (LC), welche die Domänen $V_L$ und $C_L$ umfasst, und der schweren Kette (HC), welche die Domänen $V_H$ und CH1 umfasst, gleichzeitig synthetisieren und dann in das Periplasma und schließlich in das Fermentationsmedium sekretieren. Außerhalb des Cytoplasmas erfolgt dann die Assemblierung der beiden Ketten zum funktionellen Fab-Fragment.

Für die Sekretion von rekombinanten Zielproteinen aus dem Cytoplasma in das Periplasma ist es notwendig, das 5'-Ende des ORFs des zu produzierenden Proteins *in frame* mit dem 3'-Ende einer Signalsequenz für den Protein-Export zu verknüpfen. Dazu sind prinzipiell alle Signalsequenzen geeignet, die eine Translokation mit Hilfe des Sec- oder des TAT-Apparats ermöglichen. Verschiedene Signalsequenzen sind im Stand der Technik beschrieben, so z. B. die Signalsequenzen der folgenden Gene: phoA, ompA, pelB, ompF, ompT, lamB, malE, Staphylococcal protein A, StII und andere (Choi and Lee, Appl. Microbiol. Biotechnol. 64 (2004), 625-635).

Erfindungsgemäß bevorzugt für die Sekretion von rekombinanten Zielproteinen aus dem Cytoplasma in das Periplasma ist die Signalsequenz des *phoA*- oder des ompA-Gens von *E. coli* oder die Signalsequenz für eine Cyclodextrin-Glycosyltransferase (CGTase) aus *Klebsiella pneumoniae* M5a1, oder die von dieser Signalsequenz abgeleitete Sequenz, die in US 2008/076157 A1 offenbart ist. Besonders bevorzugt ist die in EP 0 448 093 offenbarte Signalsequenz für eine CGTase aus *Klebsiella pneumoniae* M5a1, die in der vorliegenden Erfindung mit der Sequenz SEQ ID Nr. 4 angegeben ist sowie die davon abgeleitete Sequenz mit der SEQ ID Nr. 5, die auch in US 2008/076157 A1 offenbart ist.

Die Herstellung des DNS-Moleküls, das eine *in* frame-Fusion aus einer Signalsequenz und dem ORF des rekombinanten Zielproteins umfasst, erfolgt nach dem Fachmann bekannten Methoden. So kann das Gen des Zielproteins zunächst mittels PCR unter Verwendung von Oligonukleotiden als Primer amplifiziert und anschließend mit gängigen molekularbiologischen Techniken mit dem DNS-Molekül, das die Sequenz eines Signalpeptids umfasst und das auf analoge Weise wie das Gen des Zielproteins erzeugt wurde, derart verknüpft werden, dass eine *in* frame-Fusion, d.h. ein durchgehender Leserahmen umfassend die Signalsequenz und das Gen des Zielproteins, entsteht. Alternativ kann auch das gesamte DNS-Molekül, das beide oben genannten funktionellen Abschnitte umfasst, mittels Gensynthese hergestellt werden. Diese Signalsequenz-rekombinantes Gen-Fusion kann dann entweder in einen Vektor, z.B. ein Plasmid, eingebracht werden, welches dann in die Wirtszelle mittels Transformation eingebracht wird, oder direkt nach bekannten Methoden in das Chromosom der Wirtszelle integriert werden. Bevorzugt wird die Signalsequenz-rekombinantes Gen-Fusion in ein Plasmid eingebracht und die Wirtszelle wird mit diesem Plasmid transformiert.

Für die Sekretion eines aus mehreren unterschiedlichen Untereinheiten bestehenden rekombinanten Zielproteins aus dem Cytoplasma in das Periplasma ist es notwendig, die Gene aller zu produzierenden Untereinheiten (Zielgene) jeweils funktionell mit einer Signalsequenz für den Protein-Export zu verknüpfen. Dabei können die Gene der verschiedenen Untereinheiten mit der gleichen oder unterschiedlichen Signalsequenzen verknüpft werden. Bevorzugt ist die Verknüpfung mit unterschiedlichen Signalsequenzen, besonders bevorzugt ist die Verknüpfung einer Untereinheit mit der Signalsequenz des *phoA*- oder ompA-Gens von *E. coli* und die Verknüpfung einer weiteren Untereinheit mit der Signalsequenz für CGTase aus *Klebsiella pneumoniae* M5a1 mit der Sequenz SEQ ID Nr. 4 oder davon abgeleitete Sequenzen wie zum Beispiel die Sequenz SEQ ID Nr. 5.

Die Signalsequenz-Zielgen-Fusionen der einzelnen Untereinheiten können dann entweder in einen Vektor, z.B. ein Plasmid, eingebracht werden, oder direkt nach bekannten Methoden in das Chromosom der Wirtszelle integriert werden. Dabei können die Signalsequenz-Zielgen-Fusionen der einzelnen Untereinheiten auf getrennten, aber miteinander kompatiblen Plasmiden kloniert werden oder sie können auf einem Plasmid kloniert werden. Die Genfusionen können dabei in einem Operon zusammengefasst werden oder sie können in jeweils separaten Cistronen exprimiert werden. Bevorzugt ist hier ein Zusammenfassen in einem Operon. Genauso können die beiden Genkonstrukte in einem Operon zusammengefasst oder in jeweils separaten Cistronen in das Chromosom der Wirtszelle integriert werden. Bevorzugt ist auch hier ein Zusammenfassen in einem Operon.

Vorzugsweise wird das DNS-Expressionskonstrukt (Signalsequenz-Zielgen-Fusion) bestehend aus einer Signalsequenz und einem ORF codierend das zu sekretierende, rekombinante Protein, mit im gewählten Bakterienstamm funktionellen Expressionssignalen versehen (Promotor, Transkriptions-, Translationsstart, Ribosomenbindestelle, Terminator).

Als Promotoren für das Gen codierend das rekombinante Zielprotein eignen sich alle dem Fachmann bekannten Promotoren, wie einerseits beispielsweise induzierbare Promotoren wie der lac-, tac-, trc-, lambda PL, ara- oder tet-Promotor oder davon abgeleitete Sequenzen. Andererseits kann auch eine dauerhafte Expression erfolgen durch Verwendung eines konstitutiven Promotors, wie z. B. des gapA-Promotors. Es kann aber auch der normalerweise mit dem Gen des zu produzierenden rekombinanten Proteins verknüpfte Promotor Verwendung finden.

Dieses Expressionskonstrukt (Promotor-Signalsequenz-Sequenz codierend für das rekombinante Protein) wird danach unter Anwendung von dem Fachmann bekannten Methoden (z.B. Transformation) in Zellen eingebracht, die ein Lpp-Fusionsprotein bilden. Das Einbringen des Expressionskonstrukts zur Produktion des rekombinanten Proteins erfolgt z.B. auf einem Vektor, z.B. einem Plasmid wie etwa einem Abkömmling von bekannten Expressionsvektoren wie pJF118EH, pKK223-3, pUC18, pBR322, pACYC184, pASK-IBA3 oder pET. Als Selektionsmarker für Plasmide geeignet sind Gene, die für eine Resistenz gegenüber z. B. Ampicillin, Tetracyclin, Chloramphenicol, Kanamycin oder andere Antibiotika codieren.

Erfindungsgemäß wird somit vorzugsweise ein Bakterienstamm eingesetzt, in dem der ORF codierend das rekombinante Protein funktionell verknüpft mit einer Signalsequenz codierend für ein im gewählten Bakterienstamm aktives Signalpeptid, vorzugsweise ferner mit im gewählten Bakterienstamm funktionellen Expressionssignalen, vorzugsweise einem Promotor, einem Transkriptions-, Translationsstart, einer Ribosomenbindestelle, und einem Terminator versehen ist.

Ein weiterer Gegenstand der Erfindung ist ein Verfahren zur fermentativen Produktion eines rekombinanten Proteins, wobei der erfindungsgemäße Bakterienstamm in einem Fermentationsmedium kultiviert, das Fermentationsmedium nach der Fermentation von den Zellen abgetrennt und das Protein aus dem Fermentationsmedium isoliert wird.

Die Kultivierung (Fermentation) der Zellen, die ein Gen für ein Lpp-Fusionsprotein exprimieren und die ein DNS-Expressionskonstrukt bestehend aus einer Signalsequenz und einem rekombinanten Gen codierend das zu sekretierende Protein, verknüpft mit funktionellen Expressionssignalen enthalten, erfolgt nach üblichen, dem Fachmann bekannten Fermentationsverfahren in einem Bioreaktor (Fermenter).

Die Fermentation findet vorzugsweise in einem üblichen Bioreaktor, beispielsweise einem Rührkessel, einem Blasensäulen-Fermenter oder einem Airlift-Fermenter statt. Besonders bevorzugt ist ein Rührkessel-Fermenter.

Bei der Fermentation werden die Zellen des Protein-Produktionsstammes in einem Flüssigmedium über einen Zeitraum von 16-150 h kultiviert, wobei verschiedene Parameter wie z.B. die Nährstoff-Zufuhr, der Sauerstoff-Partialdruck, der pH-Wert und die Temperatur der Kultur laufend kontrolliert und genau gesteuert werden. Der Zeitraum der Kultivierung beträgt vorzugsweise 24-72 h.

Als Kulturmedien (Fermentationsmedien) kommen in Prinzip alle gängigen, dem Fachmann bekannten Medien für die Kultivierung von Mikroorganismen in Frage.

Dabei können Komplexmedien oder Minimalsalzmedien, denen ein definierter Anteil von Komplex-Komponenten wie z.B. Pepton, Trypton, Hefeextrakt, Melasse oder Corn Steep Liquor zugesetzt wird, als Medium zur Kultur der Bakterienzellen benutzt werden. Bevorzugt für die Herstellung von Pharmaproteinen sind chemisch definierte Salzmedien, also Medien, die im Gegensatz zum Vollmedium eine genau definierte Substrat-Zusammensetzung besitzen. Beispiele für geeignete Minimalsalzmedien zur Kultivierung von E. coli-Zellen sind u.a. das M9-Minimalmedium, das modifizierte Minimalmedium oder das Riesenberg Mineralmedium (Kangwa et al., 2015, AMB Expr 5, S. 70) sowie das in US 2008/0254511 A1 beschriebene Medium FM4.

Im erfindungsgemäßen Verfahren wächst ein Bakterienstamm, der ein Gen für das Lpp-Fusionsprotein exprimiert, sowie ein Gen codierend ein rekombinantes Protein, welches *in frame* mit einer Signalsequenz codierend für ein Signalpeptid verbunden ist, in einer zu einem Stamm mit Lpp-Wildtypprotein vergleichbar kurzen Fermentationszeit zu vergleichbar hohen Zelldichten heran und sekretiert dabei das rekombinante Protein in das Salzmedium.

Als primäre Kohlenstoffquelle für die Fermentation können prinzipiell alle von den Zellen verwertbaren Zucker, Zuckeralkohole oder organische Säuren bzw. deren Salze verwendet werden. Dabei werden bevorzugt Glucose, Laktose oder Glycerin eingesetzt. Besonders bevorzugt sind Glucose und Laktose. Auch ist eine kombinierte Fütterung mehrerer verschiedener Kohlenstoffquellen möglich. Die Kohlenstoffquelle kann dabei zu Beginn der Fermentation vollständig im Fermentationsmedium vorgelegt werden oder es wird nichts oder nur ein Teil der Kohlenstoffquelle zu Beginn vorgelegt und die Kohlenstoffquelle wird über den Verlauf der Fermentation zugefüttert. Besonders bevorzugt ist dabei eine Ausführungsform, bei der ein Teil der Kohlenstoffquelle vorgelegt wird und ein Teil gefüttert wird. Besonders bevorzugt wird die Kohlenstoffquelle in einer Konzentration von 10-30 g/l vorgelegt, die Fütterung gestartet, wenn die Konzentration auf kleiner 5 g/l abgefallen ist und so gestaltet, dass die Konzentration unter 5 g/l gehalten wird.

Der Sauerstoff-Partialdruck ($pO_2$) in der Kultur beträgt vorzugsweise zwischen 10 und 70 % Sättigung betragen. Bevorzugt wird ein $pO_2$ zwischen 20 und 60 %, besonders bevorzugt liegt der $pO_2$ zwischen 20 und 40 % Sättigung.

Der pH-Wert der Kultur liegt vorzugsweise zwischen pH 6 und pH 8. Bevorzugt wird ein pH-Wert zwischen 6,5 und 7,5 eingestellt, besonders bevorzugt wird der pH-Wert der Kultur zwischen 6,8 und 7,2 gehalten.

Die Temperatur der Kultur beträgt vorzugsweise zwischen 15 und 45 °C. Bevorzugt ist ein Temperaturbereich zwischen 20 und 40 °C, besonders bevorzugt ist ein Temperaturbereich zwischen 25 und 35 °C, ganz besonders bevorzugt sind 30 °C.

Bevorzugt ist das Verfahren dadurch gekennzeichnet, dass nach dem Abtrennen des Fermentationsmediums die rekombinanten Proteine aus dem Fermentationsmedium aufgereinigt werden. Die Aufreinigung von sekretierten Proteinen aus dem Rohprodukt kann durch übliche und dem Fachmann bekannte Aufreinigungsmethoden geschehen wie sie im Stand der Technik bekannt sind. Üblicherweise werden in einem ersten Schritt durch Separationsmethoden wie Zentrifugation oder Filtration die Zellen von dem sekretierten Zielprotein abgetrennt. Das Zielprotein kann dann z. B. durch Ultrafiltration aufkonzentriert werden und wird dann über Standardmethoden, wie Fällung, Chromatographie oder Ultrafiltration weiter gereinigt. Besonders bevorzugt sind hierbei Methoden, wie Affinitätschromatographie, bei der die bereits korrekt gefaltete native Konformation des Proteins ausgenützt wird.

Ein besonderer Vorteil eines Bakterienstammes exprimierend das *2xl*pp-Gen und ein rekombinantes Zielprotein liegt darin, dass die im Kulturmedium vorliegende Zielprotein-Menge höher ist als bei im Stand der Technik offenbarten Bakterienstämmen. Dies belegt Beispiel 3 (s. Tabelle 1) deutlich. Der im Kulturüberstand gemessene anti-CD154-Fab-Titer war bei Verwendung eines Bakterienstammes exprimierend 2xlppΔ (JE5512 2xlppΔ/pJF118ut-CD154) absolut gesehen (d.h. auf das gleiche Volumen normiert) fast doppelt so hoch wie bei Verwendung der bekannten lpp3-Mutante (JE5512 lpp3/pJF118ut-CD154) und betrug ein Vielfaches des für den Wildtyp-Stamm JE5512/pJF118ut-CD154 bestimmten Werts.

Auch Beispiel 4 (Tabelle 2) bestätigt, dass bei Verwendung eines Bakterienstammes exprimierend 2xlppΔ (JE5512 2xlppΔ/pCGT) absolut gesehen die höchste CGTase-Menge im Kulturüberstand gemessen wurde.

Unter erhöhter Ausbeute ist zu verstehen, dass die Ausbeute an rekombinantem Protein, das in das Kulturmedium freigesetzt wird, mindestens 1,1-mal, bevorzugt mindestens 1,5-mal und besonders bevorzugt mindestens 1,8-mal so hoch ist wie die Ausbeute an rekombinantem Protein im Kulturmedium, die nach dem gegenwärtigen Stand der Technik mit einem Wildtyp-Bakterienstamm enthaltend ein Gen für das rekombinante Protein und/oder einem Wildtyp-Bakterienstamm enthaltend ein Gen für das rekombinante Protein und exprimierend zusätzlich ein Protein zur Destabilisierung der bakteriellen Zellhülle, produziert werden kann.

Ein weiterer Vorteil eines Bakterienstammes exprimierend das 2xlpp-Gen und ein rekombinantes Zielprotein liegt darin, dass der überwiegende Teil des Zielproteins während der Kultivierung in das Fermentationsmedium ausgeschieden wird.

Dies belegt Beispiel 3 (s. Tabelle 1) wiederum deutlich. Während bei Verwendung eines Bakterienstammes exprimierend 2xlppΔ (JE5512 2xlppΔ/pJF118ut-CD154) fast 60% des anti-CD154-Fab-Titers im Kulturüberstand gemessen wurden, waren es bei Verwendung der bekannten lpp3-Mutante (JE5512 lpp3/pJF118ut-CD154) nur knapp 50 % und beim Wildtyp-Stamm JE5512/pJF118ut-CD154 nur knapp 7%.

Auch in Beispiel 4 konnten bei Verwendung eines Bakterienstammes exprimierend 2x*l*ppΔ (JE5512 2xlppΔ/pCGT) fast 60% des rekombinanten Zielproteins (CGTase) im Überstand der Kultur gemessen werden.

Unter Ausscheidung des überwiegenden Teils des rekombinanten Zielproteins in das Kulturmedium ist zu verstehen, dass bezogen auf die insgesamt produzierte Zielprotein-Menge bevorzugt über 50 Gew.-% und besonders bevorzugt über 55 Gew.-% im Kulturmedium vorliegen.

Im Gegensatz zur Verwendung von "leaky"-Bakterienstämmen haben die erfindungsgemäßen Bakterienstämme den Vorteil, dass diese stabil kultivierbar sind, **d.h.,** dass die optische Dichte der Kultur, die ein Maß für die Anzahl intakter Zellen darstellt, auch in einer späteren Kultivierungsphase nur wenig abnimmt. Dadurch ist die Protein-Produktionsphase gegenüber "leaky"-Bakterienstämmen verlängert, die Produkt-Ausbeute erhöht und es erfolgt kein Anstieg der Viskosität des Kulturmediums bedingt durch bei der Zelllyse freigesetzte DNS. Letzteres vereinfacht die anschließende Aufreinigung und Gewinnung des Zielproteins, was wiederum die Prozesskosten positiv beeinflusst.

Fig. 1 zeigt eine schematische Darstellung des unprozessierten Lpp-Fusionsproteins (A, 2xLpp) im Vergleich zum unprozessierten Lpp-Wildtypprotein (B, Lpp, Sequenz angegeben in SEQ ID Nr. 2) und des in den Beispielen verwendeten unprozessierten Lpp-Fusionsproteins (C, 2xLppΔ, Sequenz angegeben in SEQ ID Nr. 3).

Die in Figur 1 verwendeten Abkürzungen haben die folgende Bedeutung:

SP, Signalpeptid
Cys, Aminosäure Cystein
Lys, Aminosäure Lysin
Gly, Aminosäure Glycin
L, potentiell vorhandene Linkersequenz bestehend aus 0-20 Aminosäuren
Lpp, Aminosäure-Sequenz des Lpp-Wildtypproteins
Lpp(N), N-terminal lokalisierte Kopie der Aminosäuresequenz von Lpp, die bezogen auf die Lpp-Wildtyp-Sequenz an höchstens 10 Aminosäurepositionen Mutationen aufweist
Lpp(C), C-terminal lokalisierte Kopie der Aminosäuresequenz von Lpp, die bezogen auf die Lpp-Wildtyp-Sequenz an höchstens 10 Aminosäurepositionen Mutationen aufweist
Lpp(N)ΔLys$_{78}$, N-terminal lokalisierte Kopie der Aminosäuresequenz von Lpp, der bezogen auf die Lpp-Wildtyp-Sequenz die Aminosäure Lysin an Position 78 fehlt
Lpp(C)ΔCys$_{21}$, C-terminal lokalisierte Kopie der Aminosäuresequenz von Lpp, der bezogen auf die Lpp-Wildtyp-Sequenz die Aminosäure Cystein an Position 21 fehlt
-, der Strich stellt die Deletion einer Aminosäure dar

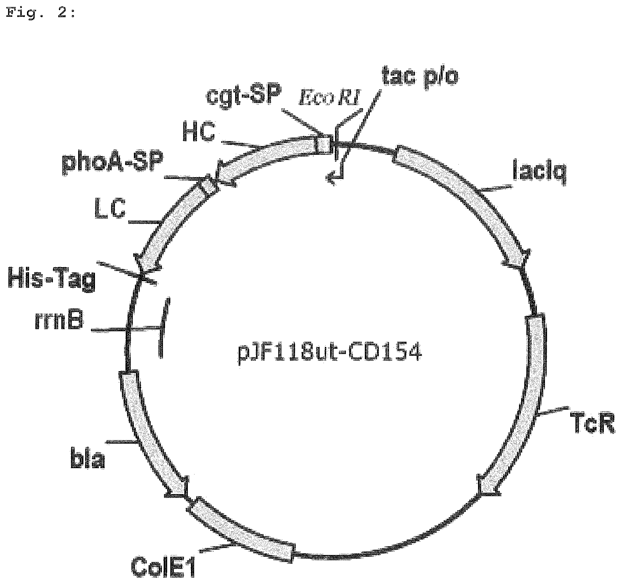

Fig. 2 zeigt eine schematische Darstellung des Expressionsplasmids pJF118ut-CD154.

Die in Figur 2 verwendeten Abkürzungen haben die folgende Bedeutung:

tac p/o:     tac-Promotor/Operator
EcoRI:        Schnittstelle des Restriktionsenzmys EcoRI
cgt-SP:       Signalpeptid der CGTase
HC:           ORF der schweren Kette des Fab-Fragments CD154
phoA-SP:     phoA-Signalpeptid
LC:           ORF der leichten Kette des Fab-Fragments CD154
His-Tag:      His-Tag am C-Terminus der leichten Kette des Fab-Fragments
rrnB:         Terminator
bla:          β-Lactamase-Gen (Ampicillin-Resistenz)
ColE1:        ColE1-Replikationsursprung
TcR:          Tetracyclin-Resistenz-Gen
lacIq:        Repressor des tac-Promotors

## Beispiele

Die folgenden Beispiele dienen der weiteren Erläuterung der Erfindung, ohne diese zu beschränken.

Sämtliche eingesetzten molekularbiologischen und mikrobiologischen Verfahren wie Polymerase-Ketten-Reaktion (PCR), Gensynthese, Isolierung und Reinigung von DNS, Modifikation von DNS durch Restriktionsenzyme, Klenow-Fragment und Ligase, Transformation, P1-Transduktion etc., wurden in der dem Fachmann bekannten, in der Literatur beschriebenen oder von den jeweiligen Herstellern empfohlenen Art und Weise durchgeführt. Die verwendeten Oligonukleotide wurden von der Firma Metabion International AG (Planegg/Deutschland) bezogen.

**Beispiel 1: Erzeugung eines *E. coli* JE5512-Stamms, der ein Lpp-Fusionsprotein (2xLppΔ-Protein) bildet (2x*lpp*Δ-Mutante)**

### 1. Herstellung des *E. coli-Stamms* JE5512 lpp::cat-sacB pKD46

Als Ausgangsstamm für die Erzeugung eines Stammes, der ein Lpp-Fusionsprotein bildet, wurde der *E. coli* lpp-Wildtyp-Stamm JE5512 (HfrC man pps) verwendet (Hirota et al. 1977, Proc. Natl. Acad. Sci. USA 74, S. 1417-1420, Stamm verfügbar vom National Institute of Genetics, Microbial Genetics Laboratory, NBRP E. coli, 1111 Yata, Mishima, Shizuoka, 411-8540 JAPAN) .

Zuerst wurde in diesem Stamm die codierende Region des chromosomalen Wildtyp-lpp-Gens (Nukleotid 1-237 in SEQ ID Nr. 1) durch eine Expressionskassette ersetzt, welche neben dem Gen für eine Chloramphenicol-Acetyltransferase (cat; UniProt No. P62577) auch das Gen für die Laevansucrase aus *B. subtilis (sacB;* UniProt No. P05655) umfasst. Zu diesem Zweck wurde als Matrize für die Amplifizierung dieser cat-sacB-Kassette mittels PCR ein Derivat des Plasmids pKO3 (Link et al. 1997, J. Bacteriol. 179, S. 6228-6237, Sequenz von pKO3 s. http://arep.med.harvard.edu/labgc/pK03v.html) verwendet, bei dem durch Schnitt mit den Restriktionsenzymen SmaI und Bst1107I und Religation des 4729 Basenpaare großen Fragments ein Großteil der Region zwischen dem cat- und dem sacB-Gen entfernt worden war. Das resultierende Plasmid wurde mit pKO3-Delta-M13 bezeichnet. Die PCR zur Amplifizierung der cat-sacB-Kassette wurde mit pKO3-Delta-M13 als Matrize und den Oligonukleotiden lpp-cat-sac-fw (SEQ ID Nr. 6) und lpp-cat-sac-rev (SEQ ID Nr. 7) durchgeführt. Dabei sind die ersten 60 Nukleotide von lpp-cat-sac-fw homolog zur 5'-seitig gelegenen Sequenz des offenen Leserahmens (ORF) von *lpp* und die ersten 60 Nukleotide von lpp-cat-sac-rev homolog zur 3'-seitig gelegenen Sequenz des lpp-ORFs. Es entstand ein lineares DNS-Fragment, das die cat-sacB-Kassette enthielt.

Der Stamm JE5512 wurde mit dem Plasmid pKD46 (Coli Genetic Stock Center CGSC#: 7739) transformiert, was zu dem Stamm JE5512 pKD46 führte. Kompetente Zellen des Stammes JE5512 pKD46, welche nach den Angaben von Datsenko und Wanner (2000, Proc. Natl. Acad. Sci. USA 97, S. 6640-6645) hergestellt worden waren, wurden mit dem linearen DNS-Fragment, das die cat-sacB-Kassette enthielt, transformiert. Die Selektion auf Integration der cat-sacB-Kassette in das Chromosom von JE5512 an die Position des Wildtyp-lpp-ORFs erfolgte auf LB-Agar-Platten, die 20 mg/l Chloramphenicol enthielten. Auf diese Weise wurden Zellen erhalten, bei denen der Wildtyp-*lpp*-ORF vollständig durch die cat-sacB-Kassette ersetzt worden war (JE5512 lpp::cat-sacB pKD46). Dass die Integration an der korrekten Position im Chromosom erfolgte, wurde mittels PCR unter Verwendung der Oligonukleotide pykF (SEQ ID Nr. 8) und ynhG2 (SEQ ID Nr. 9) und chromosomaler DNS der Chloramphenicol-resistenten Zellen als Matrize bestätigt. Zellen des Stammes JE5512 lpp::cat-sacB pKD46 exprimieren nun das Gen *cat codierend* eine Chloramphenicol-Acetyltransferase und das Gen *sacB* codierend eine Laevansucrase anstelle des lpp-Wildtyp-Gens.

### 2. Herstellung eines DNS-Fragments codierend ein 2xLppΔ-Protein

Das DNS-Fragment codierend ein 2xLppΔ-Protein wurde durch die Methode der "Overlap Extension"-PCR (Horton et al. 2013, BioTechniques 54, 129-33) hergestellt. Dazu diente zunächst chromosomale DNS von JE5512 als Matrize. Diese enthält ein Wildtyp-lpp-Gen.

Zur Erzeugung des DNS-Fragments enthaltend u.a. $lpp(N)\Delta Lys_{78}$ wurde eine PCR mit den Oligonukleotiden lpp-Allel-fw (SEQ ID Nr. 10) und lpp-2x-rev2 (SEQ ID Nr. 11) durchgeführt (PCR1). Mit dem Produkt von PCR1 als Matrize wurde eine zweite PCR mit den Oligonukleotiden lpp-Allel-fw (SEQ ID Nr. 10) und lpp-2x-rev3 (SEQ ID Nr. 12) durchgeführt (PCR2), wodurch das Produkt von PCR1 3'-seitig um 41 Basenpaare verlängert wurde.

Zur Erzeugung des DNS-Fragments enthaltend u.a. $lpp(C)\Delta Cys_{21}$ wurde eine PCR mit den Oligonukleotiden lpp-2x-fw2 (SEQ ID Nr. 13) und lpp-Allel-rev (SEQ ID Nr. 14) mit chromosomaler DNS von JE5512 als Matrize durchgeführt (PCR3). Mit dem Produkt von PCR3 als Matrize wurde eine weitere PCR mit den Oligonukleotiden lpp-2x-fw3 (SEQ ID Nr. 15) und lpp-Allel-rev (SEQ ID Nr. 14) durchgeführt (PCR4), wodurch das Produkt von PCR3 5'-seitig um 57 Basenpaare verlängert wurde.

Mit den Produkten von PCR2 und PCR4 als Matrize erfolgte schließlich eine fünfte PCR unter Verwendung der Oligonukleotide lpp-Allel-fw (SEQ ID Nr. 10) und lpp-Allel-rev (SEQ ID Nr. 14) als Primer. Das dadurch erzeugte 1005 Basenpaar lange DNS-Fragment enthielt u.a. die Signalsequenz (SP) des *lpp*-Gens in einem ORF verbunden mit einem DNS-Fragment enthaltend in diesem Beispiel die codierende Sequenz des Lpp-Wildtyp-Proteins, dem das Codon für den Lysinrest an Position 78 fehlt (bezeichnet mit $lpp(N)\Delta Lys_{78}$), einer Linkersequenz (L) bestehend in diesem Beispiel aus drei aufeinanderfolgenden Glycin-Codons sowie einem C-terminalen DNS-Fragment enthaltend in diesem Beispiel die codierende Sequenz des Lpp-Wildtyp-Proteins, dem das Codon für den Cystein-Rest an Position 21 fehlt (bezeichnet mit $lpp(C)\Delta Cys_{21}$) sowie zusätzlich jeweils noch ca. 300 Basenpaare der 5'- bzw. 3'-Region des *lpp*-Genorts (SEQ ID Nr. 16, 2x*lpp*Δ). Das von diesem DNS-Fragment gebildete 2xLppΔ-Protein ist schematisch in Fig. 1C dargestellt.

### 3. Herstellung des *E. coli-Stamms* JE5512 2xlppΔ

In einem nächsten Schritt wurde die cat-sacB-Kassette durch 2x*lpp*Δ ersetzt. Dazu wurde das Produkt aus PCR5 in kompetente Zellen des Stammes JE5512 lpp::cat-sacB pKD46 nach der Methode von Datsenko und Wanner (s.o.)

transformiert. Die Selektion auf Integration von 2x*lpp*Δ in das Chromosom von JE5512 lpp::cat-sacB pKD46 an der ursprünglichen Position des Wildtyp-lpp-Gens erfolgte auf LB-Agar-Platten inkl. 7 % Saccharose. Da nur Zellen ohne sacB-Expression auf Saccharose-haltigem Medium wachsen können, konnte auf Zellen selektiert werden, bei denen die cat-sacB-Kassette durch 2x*lpp*Δ ersetzt worden war (JE5512 2xlppΔ). Dass die Integration an der korrekten Position im Chromosom erfolgte, wurde mittels PCR unter Verwendung der Oligonukleotide pykF (SEQ ID Nr. 8) und ynhG2 (SEQ ID Nr. 9) und chromosomaler DNS der Saccharase-resistenten Zellen als Matrize bestätigt. Durch Sequenzierung des PCR-Produkts wurde die Sequenz des integrierten 2x*lpp*Δ überprüft. Der resultierende Stamm wurde mit JE5512 2xlppΔ bezeichnet.

**Beispiel 2: Erzeugung eines *E. coli* JE5512-Stamms, der ein lpp3-Allel enthält (*lpp3*-Mutante)**

Zu Vergleichszwecken wurde ausgehend vom *E. coli-Stamm* JE5512 eine *lpp*-Mutante erzeugt, die anstelle des chromosomalen *lpp*-Wildtypgens das bekannte lpp3-Allel enthält (Giam et al. 1984, Eur. J. Biochem. 141, S. 331-379). Das lpp3-Allel zeichnet sich durch eine Mutation aus, die zu dem Aminosäure-Austausch Glycin zu Asparaginsäure an Position 14 des Lpp-Proteins führt und eine gewisse "Leakiness" der Zellen für periplasmatische Proteine zur Folge hat (s. US 2008/0254511 A1).

An chromosomaler DNS der lpp3-Mutante *E. coli* "W3110 lpp3" (s. US 2008/0254511 A1 Beispiel 3) wurde eine PCR mit den Oligonukleotiden lpp-Allel-fw (SEQ ID Nr. 10) und lpp-Allel-rev (SEQ ID Nr. 14) durchgeführt. Das PCR-Produkt enthaltend das lpp3-Allel wurde analog zu 2x*lpp*Δ wie in Beispiel 1 beschrieben in das Chromosom des Stammes JE5512 lpp::cat-sacB pKD46 integriert. Die korrekte Integration des PCR-Produkts in das Chromosom wurde wie oben beschrieben mittels PCR und Sequenzierung bestätigt. Der resultierende Stamm wurde mit JE5512 lpp3 bezeichnet.

**Beispiel 3: Fermentative Produktion eines Fab-Antikörperfragments mit der 2x*lpp*Δ-Mutante im 3 1-Maßstab**

1. Herstellung des Plasmids pJF118ut-CD154

Das vorliegende Beispiel beschreibt die Produktion eines Fab-Fragments des humanisierten monoklonalen anti-CD154-Antikörpers 5c8, dessen Sequenz in Karpusas et al. (2001, Structure 9, S. 321-329) veröffentlicht ist, mit Hilfe des *E. coli* JE5512 2xlppΔ-Stamms im Vergleich zum Wildtyp-lpp-Stamm und zur lpp3-Mutante. Als Ausgangsvektor für die Klonierung und Expression der Gene des anti-CD154-Fab-Fragments diente das in US 2008/0254511 A1 beschriebene Plasmid pJF118ut. pJF118ut ist bei der DSMZ-Deutsche Sammlung von Mikroorganismen und Zellkulturen GmbH (Braunschweig) unter der Nummer DSM 18596 hinterlegt. In dieses Plasmid wurden die beiden Leserahmen für die schwere Kette (VH-CH1-Domänen) bzw. für die leichte Kette (VL-CL-Domänen) des Fab-Fragments, jeweils inklusive einer Signalsequenz, kloniert. Dazu wurde wie folgt vorgegangen: Das DNS-Fragment mit der SEQ ID Nr. 17 wurde mittels Gensynthese (Firma Eurofins Genomics) hergestellt. Dieses umfasste eine Genfusion bestehend aus

i einer von SEQ ID Nr. 5 abgeleiteten Signalsequenz und
ii dem Leserahmen für die schwere Kette des Fab-Fragments, sowie eine Genfusion bestehend aus

i der phoA-Signalsequenz von *E. coli* und
ii dem Leserahmen für die leichte Kette des Fab-Fragments und
iii einem Linker bestehend aus vier Aminosäuren C-terminal der leichten Kette und
iv einem Hexa-Histidin-Tag C-terminal des Linkers. Dieses DNS-Fragment wurde mit den Restriktionsenzymen EcoRI und PdmI geschnitten und mit dem Expressionsvektor pJF118ut, der mit EcoRI und SmaI geschnitten worden war, ligiert. Das resultierende Plasmid, bei dem die Expression der Gene für die schwere und leichte Kette des anti-CD154-Fab-Fragments unter der Kontrolle des tac-Promotors steht, wurde mit pJF118ut-CD154 bezeichnet. Fig. 2 zeigt die Plasmidkarte des Plasmids pJF118ut-CD154.

2. Produktion des anti-CD154-Fab-Antikörperfragments

Für die Produktion des anti-CD154-Fab-Antikörperfragments im Fermentermaßstab wurden die Stämme JE5512, JE5512 lpp3 und JE5512 2xlppΔ mit dem Plasmid pJF118ut-CD154 mittels der CaCl₂-Methode transformiert. Die Selektion auf plasmidhaltige Zellen erfolgte mittels Tetracyclin (20 mg/l).

Die Produktion wurde in 3 l Rührkesselfermentern durchgeführt.

1,2 l eines für die Kultivierung von *E. coli* üblichen Mineralsalzmediums inkl. 15 g/l Glukose, angereichert mit Komplexkomponenten (1,5 g/l Hy-Express II (Kerry); 1,0 g/l Amisoy (Kerry); 0,5 g/l Hy-Yest (Kerry)), wurden mit einer Vorkultur, die im Schüttelkolben ca. 6 h in einem Komplexmedium (30 g/l Phytone Peptone (BD Biosciences), 5 g/l Hefeetrakt (Oxoid), 5 g/l NaCl) bei 30 °C kultiviert worden war, ungefähr auf eine $OD_{600}$ = 0,01 angeimpft. Das Animpfen stellt den Zeitpunkt 0 der Fermentation bzw. den Fermentationsbeginn dar. Während der Fermentation wurde eine Temperatur von 30 °C eingestellt und der pH-Wert durch Zudosierung von NH$_4$OH bzw. H$_3$PO$_4$ bei einem Wert von 7,0 konstant gehalten. Die Kultur wurde zu Beginn mit 400 rpm gerührt und mit 2 vvm einer über einen Sterilfilter entkeimten Druckluft begast. Unter diesen Startbedingungen war die Sauerstoff-Sonde vor der Inokulation auf 100% Sättigung kalibriert worden. Der Soll-Wert für die O$_2$-Sättigung während der Fermentation wurde auf 30 % eingestellt. Nach Absinken der O$_2$-Sättigung unter den Soll-Wert wurde eine Regulationskaskade gestartet, um die O$_2$-Sättigung wieder an den Soll-Wert heranzuführen. Dabei wurde zunächst die Gaszufuhr kontinuierlich auf max. 5 vvm erhöht und anschließend die Rührgeschwindigkeit auf max. 1.500 rpm kontinuierlich gesteigert. Die Fütterung mit Glukose wurde 10 h nach Kultivierungsbeginn gestartet. Etwa 0,5-1 h vor der geplanten Induktion wurde die Temperatur von 30 °C auf 27 °C abgesenkt. Die Induktion der Expression erfolgte durch Zugabe von Isopropyl-β-D-thiogalactopyranosid (IPTG) ad 0,1 mM nach ca. 21-23 h Kultivierungsdauer.

Nach 64 h Kultivierungsdauer wurden Proben entnommen, die Zellen durch Zentrifugation vom Kulturmedium abgetrennt und der Gehalt des Fab-Fragments im Kulturüberstand mit Hilfe eines Sandwich-ELISA-Tests bestimmt (s.u.). Zur Ermittlung der Menge des Zielproteins in der gesamten Kulturbrühe, d.h. der Summe aus intra- und extrazellulär vorhandenem Fab-Fragment, wurde der Fab-Gehalt in der homogenisierten Kulturbrühe bestimmt. Dazu wurden 150 μl Kulturbrühe mit 850 μl 100 mM Tris/Cl-Puffer (pH 7,4) vermischt und die Zellen mit einem FastPrep-Homogenisator (FastPrep-24™ 5G, MP Biomedicals) aufgeschlossen. Nach Abtrennung der Zelltrümmer durch Zentrifugation wurde der klare Überstand in den Sandwich-ELISA-Test eingesetzt.

Die Quantifizierung des anti-CD154-Fab-Fragments erfolgte über einen dem Fachmann bekannten Sandwich-ELISA-Test. Dabei diente ein immobilisierter Anti-Fd-Schwere-Kette-Antikörper (The Binding Site, Produktnummer: PC075) als Fänger und ein Peroxidase-konjugierter goat Anti-Human Kappa Light Chains Antikörper (Sigma, Produktnummer: A7164) als Detektions-Antikörper. Die Quantifizierung erfolgte durch Umsetzung des chromogenen Substrats Dako TMB+ (Dako, Produktnummer: S1599) durch die Peroxidase und die damit einhergehende Absorptionsänderung bei 450 nm. Zur Kalibrierung des ELISA wurde das Fab-Fragment "Human Fab/Kappa" (Bethyl Laboratories, Produktnummer: P80-115) verwendet.

In Tabelle 1 sind die Ausbeuten des anti-CD154-AntikörperFragments im Kulturüberstand und der Gesamtkultur aufgelistet.

**Tabelle 1:** anti-CD154-Titer in Kulturüberstand und Gesamtkultur nach 64 h Fermentation

|  | anti-CD154-Fab (g/l) | |
| --- | --- | --- |
| **Stamm** | **Überstand** | **Gesamtkultur** |
| JE5512 / pJF118ut-CD154 | 0,04 | 0,59 |
| JE5512 lpp3 / pJF118ut-CD154 | 0,95 | 1,95 |
| JE5512 2xlppΔ / pJF118ut-CD154 | 1,70 | 2,90 |

**Beispiel 4: Fermentative Produktion einer Cyclodextrin-Glycosyl-Transferase mit der 2x*lpp*Δ-Mutante im 3 1-Maßstab**

Für die Produktion einer Cyclodextrin-Glycosyl-Transferase (CGTase) im 3 1-Maßstab wurden die Stämme JE5512, JE5512 lpp3 und JE5512 2xlppΔ mit dem Plasmid pCGT mittels der CaCl$_2$-Methode transformiert. Die Selektion auf plasmidhaltige Zellen erfolgte mittels Tetracyclin (20 mg/l).

Die Herstellung des Plasmids pCGT für die Überexpression der CGTase ist in Beispiel 4 von US 2008/0254511 A1 beschrieben, die Plasmidkarte in Fig. 4 von US 2008/0254511 A1 angegeben. Im Wesentlichen enthält das Plasmid neben dem Gen für die Resistenz gegen Tetracyclin u.a. auch das Strukturgen der CGTase aus *Klebsiella pneumoniae* M5a1 einschließlich der nativen CGTase-Signalsequenz. Die Expression des CGTase codierenden Gens steht unter der Kontrolle des tac-Promotors.

Die Kultivierung zur fermentativen Produktion der CGTase mit den Stämmen JE5512/pCGT, JE5512 lpp3/pCGT und JE5512 2xlppΔ/pCGT erfolgte wie in Beispiel 3 beschrieben.

Nach 64 h Fermentationsdauer wurden Proben entnommen und anschließend der CGTase-Gehalt im Kulturüberstand und der homogenisierten und geklärten Kulturbrühe (s. Beispiel 3) anhand der Menge an enzymatisch aus Stärke produziertem Cyclodextrin (CD) durch einen CGTase-Aktivitätstest bestimmt.

**CGTase-Aktivitätstest**

| Testpuffer: | 5 mM Tris-HCl-Puffer, 5 mM CaCl$_2$ x 2 H$_2$O, pH 6,5 |
| Substratlösung: | 10 %ige Stärkelösung (Merck Nr.1.01252) in Testpuffer, pH 6,5 |
| Testansatz: | 0,2 ml Substratlösung + 0,2 ml entsprechend verdünnte CGTase-Probe (Kulturüberstand bzw. homogenisierte und geklärte Kulturbrühe) |
| Reaktionstemperatur: | 40°C |

Enzymtest:

* Vortemperieren von Substratlösung und CGTase-haltiger Probe (ca. 5 min bei 40°C)
* Herstellen des Testansatzes durch rasches Mischen (Whirl-Mixer) von Substratlösung und CGTase-haltige Probe, wobei die Probe gegebenenfalls mit Testpuffer verdünnt wird, so dass in der anschließenden HPLC-Analytik ein Wert von 0,9-1,5 g/l CD bestimmt wird;
* Inkubation für 3 min bei 40°C
* Stoppen der Enzymreaktion durch Zugabe von 0,6 ml Methanol, rasches Mischen (Whirl-Mixer)
* Abkühlen des Ansatzes auf Eis (ca. 5 min)
* Abzentrifugieren (5 min, 12 000 rpm) und Abpipettieren des klaren Überstandes
* Analyse der Menge an produziertem CD mittels HPLC: Die Analyse wurde an einem Agilent HP 1100 HPLC-System mit einer Nucleodur 100-3 NH2-RP Säule (150 mm x 4,6 mm, Macherey-Nagel) und 64% Acetonitril in Wasser(v/v)als Fließmittel, bei einer Flussrate von 2,1 ml/min durchgeführt. Die Detektion erfolgte über einen RI-Detektor (1260 Infinity RI, Agilent) und die Quantifizierung wurde anhand der Peakfläche und einem □-CD-Standard (Cavamax W6-8 Pharma, Wacker) vorgenommen.

Berechnung der Enzymaktivität:

$$A = G*V1*V2/(t*MG)\ [U/ml]$$

A = Aktivität,
G = Gehalt an CD in mg/l
V1 = Verdünnungsfaktor im Testansatz
V2 = Verdünnungsfaktor der CGT-haltigen Probe vor dem Einsatz im Test; wenn unverdünnt gilt: V2 = 1
t = Reaktionszeit in min
MG = Molekulargewicht in g/mol (MG$_{CD}$ = 973 g/mol)
1 Unit (U) $\triangleq$ 1 $\mu$mol/l Produkt (CD) / min

Tabelle 2 zeigt die jeweils erzielten CGTase-Ausbeuten.

**Tabelle 2:** CGTase-Ausbeute im Kulturüberstand nach 64 h Fermentation

| Stamm | CGTase (U/ml) |
|---|---|
| JE5512 / pCGT | 28 |
| JE5512 lpp3 / pCGT | 540 |
| JE5512 2xlppΔ / pCGT | 619 |

Der Anteil der von JE5512 2xlppΔ / pCGT in das Medium freigesetzten CGTase lag im Vergleich zum Gesamtprotein - ähnlich wie bei dem anti-CD154-Fab - bei ca. 58 %.

In den Beispielen 3 und 4 ist die fermentative Produktion eines medizinisch relevanten Fab-Antikörperfragments bzw. eines technischen Enzyms mit verschiedenen E. coli-Stämmen dargestellt. Dabei zeigt sich in beiden Fällen die erfindungsgemäße 2xlppΔ-Mutante im Vergleich zu einem lpp-Wildtypstamm sowie einer lpp3-Mutante mit "leaky-Phänotyp" im Blick auf die in das Kulturmedium freigesetzte Menge des Zielproteins überlegen.

**Patentansprüche**

1. Bakterienstamm der Art *Escherichia coli* enthaltend mindestens ein Gen codierend für ein rekombinantes Protein **dadurch gekennzeichnet, dass** er
einen offenen Leserahmen bestehend aus

> i einem DNS-Fragment codierend ein N-terminales Signalpeptid, das die Translokation des Proteins in das Periplasma vermittelt,

>> wobei die Aminosäuresequenz des Signalpeptids des Lpp-Fusionsproteins identisch zur Aminosäuresequenz des Signalpeptids des Wildtyp-Lpp-Proteins (SEQ ID Nr. 2 von Aminosäure 1 bis 20) ist oder
>> das N-terminale Signalpeptid an Aminosäure-Position 14 statt Glycin eine andere proteinogene Aminosäure enthält und an allen anderen Aminosäure-Positionen identisch zum Signalpeptid des Wildtyp-Lpp-Proteins ist oder
>> wobei es sich beim N-terminalen Signalpeptid um ein Signalpeptid der Lipoproteine Pal, NlpI, NlpB oder OsmB von *Escherichia coli* handelt,
>> verknüpft mit

> ii einer darauffolgenden DNS-Sequenz *(lpp(N))* codierend ein Lipoprotein (Lpp(N)), wobei die von lpp(N) codierte Aminosäuresequenz die Aminosäuresequenz des Wildtyp-Lpp-Proteins (SEQ ID Nr. 2 von Aminosäure 21 bis 78) ist oder sich nur dadurch von dieser unterscheidet, dass in Lpp(N) die im Wildtyp-Lpp-Protein vorhandene C-terminale Aminosäure Lysin mutiert ist und
> iii einer weiteren DNS-Sequenz *(lpp(C))* codierend ein Lipoprotein (Lpp(C)), wobei die von lpp(C) codierte Aminosäuresequenz die Aminosäuresequenz des Wildtyp-Lpp-Proteins ist oder sich von dieser nur dadurch unterscheidet, dass in Lpp(C) die im Wildtyp-Lpp-Protein vorhandene N-terminale Aminosäure Cystein mutiert ist,

> enthält.

2. Bakterienstamm gemäß Anspruch 1, **dadurch gekennzeichnet, dass** dieser kein weiteres Gen enthält, das ein Protein mit einer Identität von mindestens 80% im Vergleich zu SEQ ID Nr. 2 von Aminosäure 21 bis 78 codiert.

3. Bakterienstamm gemäß einem oder mehreren der Ansprüche 1 bis 2, **dadurch gekennzeichnet, dass** Lpp(N) und Lpp(C) über einen Linker bestehend aus einer bis mehreren Aminosäuren verbunden sind.

4. Bakterienstamm gemäß einem oder mehreren der Ansprüche 1 bis 3, **dadurch gekennzeichnet, dass** sich die von *lpp(N)* und *lpp(C)* codierten Aminosäuresequenzen dadurch von SEQ ID Nr. 2 von Aminosäure 21 bis 78 unterscheiden, dass

> Lpp(N) die in SEQ ID Nr. 2 von Aminosäure 21 bis 78 vorhandene C-terminale Aminosäure Lysin fehlt oder
> Lpp(C) die in SEQ ID Nr. 2 von Aminosäure 21 bis 78 vorhandene N-terminale Aminosäure Cystein fehlt.

5. Bakterienstamm gemäß einem oder mehreren der Ansprüche 1 bis 4, **dadurch gekennzeichnet, dass** es sich bei der proteinogenen Aminosäure an Position 77 in mindestens einer der von *lpp(N)* oder *lpp(C)* codierten Aminosäuresequenzen um Cystein handelt.

6. Bakterienstamm gemäß einem oder mehreren der Ansprüche 1 bis 5, **dadurch gekennzeichnet, dass** es sich bei der proteinogenen Aminosäure an Position 14 des N-terminalen Signalpeptids um Asparaginsäure handelt.

7. Bakterienstamm gemäß einem oder mehreren der Ansprüche 1 bis 6, **dadurch gekennzeichnet, dass** die Aminosäuren, die die Linkersequenz bilden, ausgewählt sind aus der Gruppe bestehend aus Glycin, Serin und Alanin.

8. Bakterienstamm gemäß einem oder mehreren der Ansprüche 1 bis 7, **dadurch gekennzeichnet, dass** der offene Leserahmen codierend das 2xLpp-Protein im Chromosom anstelle der in SEQ ID Nr. 1 angegebenen Sequenz lokalisiert ist.

9. Bakterienstamm gemäß einem oder mehreren der Ansprüche 1 bis 8, **dadurch gekennzeichnet, dass** das rekombinante Protein ein heterologes Protein ist.

10. Verfahren zur fermentativen Produktion eines rekombinanten Proteins **dadurch gekennzeichnet, dass** ein Bakterienstamm gemäß einem oder mehreren der Ansprüche 1 bis 9 in einem Fermentationsmedium kultiviert, das Fermentationsmedium nach der Fermentation von den Zellen abgetrennt und das Protein aus dem Fermentationsmedium isoliert wird.

11. Verfahren gemäß Anspruch 10, **dadurch gekennzeichnet, dass** nach dem Abtrennen des Fermentationsmediums die rekombinanten Proteine aus dem Fermentationsmedium aufgereinigt werden.

**Claims**

1. Bacterial strain of the species Escherichia coli containing at least one gene encoding a recombinant protein, **characterized in that** it
   contains an open reading frame consisting of

   i a DNA fragment encoding an N-terminal signal peptide which mediates the translocation of the protein into the periplasm,

   wherein the amino acid sequence of the signal peptide of the Lpp fusion protein is identical to the amino acid sequence of the signal peptide of the wild-type Lpp protein (SEQ ID No. 2 from amino acid 1 to 20) or the N-terminal signal peptide contains a proteinogenic amino acid other than glycine at amino acid position 14 and is identical to the signal peptide of the wild-type Lpp protein at all other amino acid positions or wherein the N-terminal signal peptide is a signal peptide of the lipoproteins Pal, NlpI, NlpB or OsmB of Escherichia coli,
   linked to

   ii a following DNA sequence (lpp(N)) encoding a lipoprotein (Lpp(N)), wherein the amino acid sequence encoded by lpp(N) is the amino acid sequence of the wild-type Lpp protein (SEQ ID No. 2 from amino acid 21 to 78) or only differs therefrom **in that** the C-terminal amino acid lysine present in the wild-type Lpp protein is mutated in Lpp(N) and
   iii a further DNA sequence (lpp(C)) encoding a lipoprotein (Lpp(C)), wherein the amino acid sequence encoded by lpp(C) is the amino acid sequence of the wild-type Lpp protein or only differs therefrom **in that** the N-terminal amino acid cysteine present in the wild-type Lpp protein is mutated in Lpp(C).

2. Bacterial strain according to Claim 1, **characterized in that** it does not contain a further gene which encodes a protein having an identity of at least 80% in comparison with SEQ ID No. 2 from amino acid 21 to 78.

3. Bacterial strain according to either or both of Claims 1 and 2, **characterized in that** Lpp(N) and Lpp(C) are connected via a linker consisting of one to more than one amino acid.

4. Bacterial strain according to one or more of Claims 1 to 3, **characterized in that** the amino acid sequences encoded by lpp(N) and lpp(C) differ from SEQ ID No. 2 from amino acid 21 to 78 **in that**

   Lpp(N) lacks the C-terminal amino acid lysine present in SEQ ID No. 2 from amino acid 21 to 78 or
   Lpp(C) lacks the N-terminal amino acid cysteine present in SEQ ID No. 2 from amino acid 21 to 78.

5. Bacterial strain according to one or more of Claims 1 to 4, **characterized in that** the proteinogenic amino acid at position 77 in at least one of the amino acid sequences encoded by lpp(N) or lpp(C) is cysteine.

6. Bacterial strain according to one or more of Claims 1 to 5, **characterized in that** the proteinogenic amino acid at position 14 of the N-terminal signal peptide is aspartic acid.

7. Bacterial strain according to one or more of Claims 1 to 6, **characterized in that** the amino acids which form the linker sequence are selected from the group consisting of glycine, serine and alanine.

8. Bacterial strain according to one or more of Claims 1 to 7, **characterized in that** the open reading frame encoding the 2xLpp protein is located in the chromosome instead of the sequence specified in SEQ ID No. 1.

9. Bacterial strain according to one or more of Claims 1 to 8, **characterized in that** the recombinant protein is a heterologous protein.

10. Method for fermentative production of a recombinant protein, **characterized in that** a bacterial strain according to one or more of Claims 1 to 9 is cultured in a fermentation medium, the fermentation medium is removed from the cells after the fermentation, and the protein is isolated from the fermentation medium.

11. Method according to Claim 10, **characterized in that** the recombinant proteins are purified from the fermentation medium after the removal of the fermentation medium.

**Revendications**

1. Souche bactérienne de l'espèce Escherichia coli contenant au moins un gène codant pour une protéine recombinante, **caractérisée en ce qu'**elle contient
un cadre de lecture ouvert constitué par

> i un fragment d'ADN codant pour un peptide signal N-terminal qui intervient dans la translocation de la protéine dans le périplasme,

>> la séquence d'acides aminés du peptide signal de la protéine de fusion Lpp étant identique à la séquence d'acides aminés du peptide signal de la protéine Lpp de type sauvage (acides aminés 1 à 20 de la séquence SEQ ID NO. 2) ou
>> le peptide signal N-terminal contenant au niveau de la position d'acide aminé 14 un acide aminé protéinogène différent au lieu de la glycine et étant identique au niveau de toutes les autres positions d'acide aminé au peptide signal de la protéine Lpp de type sauvage ou
>> le peptide signal N-terminal étant un peptide signal des lipoprotéines Pal, NlpI, NlpB ou OsmB d'Escherichia coli, lié à

> ii une séquence d'ADN consécutive (lpp(N)) codant pour une lipoprotéine (Lpp(N)), la séquence d'acides aminés codée par lpp(N) étant la séquence d'acides aminés de la protéine Lpp de type sauvage (acides aminés 21 à 78 de la séquence SEQ ID NO. 2) ou n'en différant que par le fait que dans Lpp(N), l'acide aminé C-terminal lysine présent dans la protéine Lpp de type sauvage est muté et
> iii une autre séquence d'ADN (lpp(C)) codant pour une lipoprotéine (Lpp(C)), la séquence d'acides aminés codée par lpp(C) étant la séquence d'acides aminés de la protéine Lpp de type sauvage ou n'en différant que par le fait que dans Lpp(C), l'acide aminé N-terminal cystéine présent dans la protéine Lpp de type sauvage est muté.

2. Souche bactérienne selon la revendication 1, **caractérisée en ce qu'** elle ne contient pas d'autre gène codant pour une protéine présentant une identité d'au moins 80% par rapport aux acides aminés 21 à 78 de la séquence SEQ ID NO. 2.

3. Souche bactérienne selon l'une ou plusieurs des revendications 1 à 2, **caractérisée en ce que** Lpp(N) et Lpp(C) sont reliés par l'intermédiaire d'un lieur constitué d'un à plusieurs acides aminés.

4. Souche bactérienne selon l'une ou plusieurs des revendications 1 à 3, **caractérisée en ce que** les séquences d'acides aminés codées par lpp(N) et lpp(C) diffèrent des acides aminés 21 à 78 de la séquence SEQ ID NO. 2 **en ce que**

> dans Lpp(N), l'acide aminé C-terminal lysine présent dans les acides aminés 21 à 78 de la séquence SEQ ID NO 2 fait défaut ou
> dans Lpp(C), l'acide aminé N-terminal cystéine présent dans les acides aminés 21 à 78 de la séquence SEQ ID NO 2 fait défaut.

5. Souche bactérienne selon l'une ou plusieurs des revendications 1 à 4, **caractérisée en ce que** l'acide aminé protéinogène en position 77 dans au moins l'une des séquences d'acides aminés codées par lpp(N) ou lpp(C) est la cystéine.

6. Souche bactérienne selon l'une ou plusieurs des revendications 1 à 5, **caractérisée en ce que** l'acide aminé protéinogène en position 14 du peptide signal N-terminal est l'acide aspartique.

7. Souche bactérienne selon l'une ou plusieurs des revendications 1 à 6, **caractérisée en ce que** les acides aminés formant la séquence de lieur sont choisis dans le groupe constitué par la glycine, la sérine et l'alanine.

8. Souche bactérienne selon l'une ou plusieurs des revendications 1 à 7, **caractérisée en ce que** le cadre de lecture ouvert codant pour la protéine 2xLpp est localisé dans le chromosome à la place de la séquence indiquée dans la séquence SEQ ID NO. 1.

9. Souche bactérienne selon l'une ou plusieurs des revendications 1 à 8, **caractérisée en ce que** la protéine recombinante est une protéine hétérologue.

10. Procédé pour la production par fermentation d'une protéine recombinante, **caractérisé en ce qu'**on cultive une souche bactérienne selon l'une ou plusieurs des revendications 1 à 9 dans un milieu de fermentation, on sépare le milieu de fermentation des cellules après la fermentation et on isole la protéine à partir du milieu de fermentation.

11. Procédé selon la revendication 10, **caractérisé en ce que**, après séparation du milieu de fermentation, les protéines recombinantes sont purifiées à partir du milieu de fermentation.

Fig. 1: Schematische Darstellung des unprozessierten Lpp-Fusionsproteins (A), des unprozessierten Lpp-Wildtypproteins (B)
und des in den Beispielen verwendeten unprozessierten Lpp-Fusionsproteins 2xLppΔ (C).

A)

| SP | Lpp(N) | L | Lpp(C) |

B)

| SP | Cys | | Lys |

Lpp

C)

| SP | Cys | | Gly3 | | Lys |

Lpp(N) ΔLys$_{78}$          Lpp(C) ΔCys$_{21}$

Fig. 2:

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080254511 A1
- US 5223482 A
- EP 2204441 A1
- CN 102827860 B
- US 2008076157 A1
- EP 0448093 A


**In der Beschreibung aufgeführte Nicht-Patentliteratur**

- **INOUYE et al.** *J. Bact.*, 1977, vol. 132, 308-313
- **SUZUKI**. *Mol. Gen. Genet.*, 1978, vol. 167, 1-9
- **GIAM et al.** *Eur. J. Biochem.*, 1984, vol. 141, 331-379
- **ZÜCKERT**. *Biochimica et Biophysica Acta*, 2014, vol. 1843, 1509-16
- **GIAM et al.** *J Biol Chem*, 1984, vol. 259, 5601-5
- **CHANG et al.** *J Biol Chem*, 2012, vol. 287, 418-28
- **SHA et al.** *Infect Immun*, 2008, vol. 76, 1390-409
- **GIAM et al.** *J. Biol. Chem.*, 1984, vol. 259, 5601-5605
- **HAYASHI** ; **WU**. *J. Bioenerg. Biomembr.*, 1990, vol. 22, 451-471
- **HORTON et al.** *BioTechniques*, 2013, vol. 54, 129-133
- **LINK et al.** *J. Bacteriol.*, 1997, vol. 179, 6228-6237
- **SUN et al.** *Appl. Environ. Microbiol.*, 2008, vol. 74, 4241-4245
- **DATSENKO** ; **WANNER**. *Proc. Natl. Acad. Sci. U S A.*, vol. 97, 6640-6645
- **CHOI** ; **LEE**. *Appl. Microbiol. Biotechnol*, 2004, vol. 64, 625-635
- **KANGWA et al.** *AMB Expr*, 2015, vol. 5, 70
- **HIROTA et al.** Proc. Natl. Acad. Sci. USA. Microbial Genetics Laboratory, 1977, vol. 74, 1417-1420
- **DATSENKO** ; **WANNER**. *Proc. Natl. Acad. Sci. USA*, 2000, vol. 97, 6640-6645
- **HORTON et al.** *BioTechniques*, 2013, vol. 54, 129-33
- **KARPUSAS et al.** *Structure*, 2001, vol. 9, 321-329
- Human Fab/Kappa. Bethyl Laboratories